# EUROPEAN PATENT SPECIFICATION

(11) **EP 2 296 541 B1**
(45) Date of publication and mention of the grant of the patent: **10.04.2013**
(21) Application number: 09707308.4
(22) Date of filing: 04.02.2009
(51) Int. Cl.: A61B 5/04, G03F 7/00

(54) **METALLIZATION PROCESS TO OBTAIN A MICROELECTRODE ON A PHOTOPATTERNABLE SUBSTRATE AND ITS BIOMEDICAL APPLICATION ON AN ORGAN TRANSPLANT MONITORING DEVICE**
METALLISIERUNGSVERFAHREN ZUR HERSTELLUNG EINER MIKROELEKTRODE AUF EINEM FOTOSTRUKTURIERBAREN SUBSTRAT UND SEINE BIOMEDIZINISCHE ANWENDUNG IN EINEM ORGANTRANSPLANTAT-ÜBERWACHUNGSGERÄT
PROCÉDÉ DE MÉTALLISATION POUR OBTENIR UNE MICROÉLECTRODE SUR UN SUBSTRAT SUR LEQUEL DES MOTIFS PEUVENT ÊTRE FORMÉS PAR PHOTOEXPOSITION ET SON APPLICATION BIOMÉDICALE SUR UN DISPOSITIF DE SURVEILLANCE DE TRANSPLANTATION D'ORGANE

(30) Priority: 05.02.2008 EP 08380028
(43) Date of publication of application: 23.03.2011
(73) Proprietor: Ikerlan, S. Coop, 20500 Arrasate-Mondragon (ES); I2M-Design, 08193 Cerdanyola del Valles (ES)
(72) Inventor: BLANCO BARRO, Francisco Javier, E-18760 Madrid (ES); TIJERO SERNA, Maria, Aretxabaleta 20550 Gipuzkoa (ES); FERNANDEZ LEDESMA, Luis Jose, E-50500 Zaragoza (ES); AGUILO LLOBET, Jordi, E-08190 Sant Cugat (ES); VILLA SANZ, Rosa, E-08015 Barcelona (ES); CALDERON, Enric, 08172 - Sant Cugat del Valles (ES); GOMEZ SUAREZ, Cristina, E-28230 Majadahonda-Madrid (ES); GABRIEL BUGUÑA, Gemma, E-08027 Barcelona (ES)
(74) Representative: Ponti Sales, Adelaida
(86) International application number: PCT/EP2009/051289
(87) International publication number: WO 2009/098242

(56) References cited:
- WO-A-2005/117554
- WO-A-2006/069323
- US-A1- 2005 277 064
- US-B1- 6 198 965

## Description

### Field of the invention

The present invention is related to a metallization process to obtain a microelectrode on a photopatternable substrate and its biomedical application on an organ transplant monitoring device.

### Background

The state of the art refers several sensor microdevices for biomedical applications. The Spanish patent ES-2154241-A1 discloses silicium based multisensors of millimetric size, needle shaped. One end, the head, is larger. The sensor is located in the narrow part whereas the signal treatment and modulation circuits are placed in the widest, proximal end.

A further microfabricated neural probe and method making it is disclosed in WO 2005/117554.

These multisensor microdevices are aimed to continuously and simultaneously monitor impedance changes, ionic concentration, such as extracellular K+, pH or temperature of tissues, organs or body fluids.

The impedance or electric bioimpedance as such cannot be used directly as a physiological parameter. However, bioimpedance values or the passive electric properties derived from reflecting indirectly several states and events relevant to the medical practice.

The impedance has been used to detect the presence and state of several living tissue pathologies, such as cancer (US2006100488, US2005065418, EP1600104, WO03084383, US2003105411), ischemia (WO2004105862, US5807272, WO0114866) or even caries (US6230050).

Generally, the cellular membrane of living beings behaves like a shallow layer of imperfect dielectric material that separates two electrolytic means (intracellular and extracellular). This model explains common characteristics of living tissues in terms of impedance in relation with other materials. Any factor that modifies any of those parameters of this model will have an impact on some of the values obtained through electrical impedance. Several cellular and histological alterations modifying impedance values have been described, as for example: cellular edema, extracellular edema, necrosis, closing or opening of intercellular ionic unions, physical uncoupling of cells, ionic imbalance, cell morphological changes and opening of pores in the membrane by means of electroporation.

For each frequency analyzed, the electrical impedance provides two values. Said two values can be expressed as the "magnitude" and "phase" of the impedance. However, other pairs of values mathematically related to said magnitude and phase can be used as well (e.g. real and imaginary parts of the impedance or "conductivity and permittivity"). All these values, taken individually or mathematically combined, can be used for tissue characterization. For minimizing the number of values needed for tissue characterization of living animals, the use of mathematical models based on the experience (e.g. Cole model) is frequent.

Several techniques for obtaining passive electrical properties of living tissue exist. Generally, for high frequencies (>100 MHz) methods based in the transmission or reflection of electromagnetic waves are used. For lower frequencies, methods based in inductive coupling can be used, although methods based on measuring by means of electrodes are much more common. In many cases, the measuring is carried out by means of four electrodes (Tetrapolar electrical impedance methods or Kelvin methods), but, however, measures using two or three electrodes are possible as well. Measuring techniques of bioimpedances and its applications are widely described in the book "Bioimpedance and Bioelectricity Basics" by S. Grimnes and O. G. Martinsen ,Academic Press (London, UK), 2000.

Thus, US 6,198,965 describes a telemetry system for monitoring a rejection reaction of a transplanted organ transplanted within a patient's body. The telemetry system includes (a) a telemetry control unit located outside the body of the patient; and (b) a telemetry monitoring unit implanted within the body of the patient.

On the other hand, WO 2006/069323 describes implantable hermetically sealed structures and methods for making the same. Also provides devices, systems and kits including the hermetically sealed structure, as well as methods of using such devices and systems. The miniaturized, corrosion resistant hermetic package provides protection for an effector, e.g. that includes an integrated circuit (IC) in long term contact with saline, blood or other body fluid. The integrated circuit refers to a tiny complex of electronic components and their connections that is produced in or on a small slice of material, i.e., chip, such as a silicon chip.

In spite of ultra-pure crystal Silicium (Si) being the material used par excellence in microelectronics and, therefore, the most widely used for the development of microsystems, it shows many backdraws. Its relative fragility makes extraordinarily difficult its manipulation in clinical applications and its introduction and/or implant in living beings can damage them.

Moreover, the Si substrate resistivity distorts the electrical records at certain frequencies, since the applied currents used for the record can partially leak out through the substrate. This phenomenon also reduces the effectiveness of the techniques necessary for obtaining the measuring electrodes, such as platinization by means of an electromigration process.

On the other hand, because of the motions during surgery manipulation of internal organs for a correct surgery, the inserted needle breaks easily and often in the linking zone with the non-inserted part of the needle, where the connections are arranged. This causes the whole encapsulated system to be discarded, together with the data transmission electronics, therefore involving a high cost, as well as the necessity to recover the fragment that is left in the tissue, which extraction can cause hepatic injuries.

Therefore, the fragility of a needle obtained from a Silicium substrate according to the state of the art is even more problematic for its application in organ transplant.

Besides, the different biocompatible materials described in the state of the art, such as polyamide, BCB or parilene polymers, show drawbacks related to the flexibility and bending during insertion, and therefore they do not penetrate the tissue suitably in applications related to organ transplant.

It has not been described yet in the state of the art a technology for obtaining a microelectrode with such a thickness that gives it a rigidity enough so as it can be inserted in an organ without being broken during its utilization in transplants and, thus, without causing injuries in the organ, and at the same time, flexible enough to be handled in motion and inserted without bending.

Photolitography is the most widely used technology for the manufacturing of sensor elements or electrodes in polymeric substrates with high resolution. This technique consist of the spinning of a photopatternable polymer, followed by a selective exposure by using a mask pattern placed onto, thus allows light passing through the desired areas. In this way, the polymer is removed from the areas where the metal is to be deposited by means of a subsequent development process. However, the thickness obtained in polymeric materials is of the order of microns, very lower than the value necessary to ensure enough rigidity for its correct insertion in an organ without bending.

Other techniques, such as "screen printing", can be applied on substrates with high roughness. This technique consists of depositing a paste compound through a material stainless steel mask. The paste consists of a mixture of the selected material, organic adhesive and a solvent. The desired pattern is transferred to the substrate by forcing the paste passing through the mask openings. However, with this technique it is not possible to obtain tracks with a high resolution.

### Brief description of the invention

Therefore, the present invention object is to overcome the problems of the previous techniques providing a new metallization technique that can be used to obtain a microelectrode with sensors suitable to organ transplant.

### Brief description of the drawings

Fig. 1 shows a scheme with the steps of the metallization process of a photopatternable polymer according to the present invention.

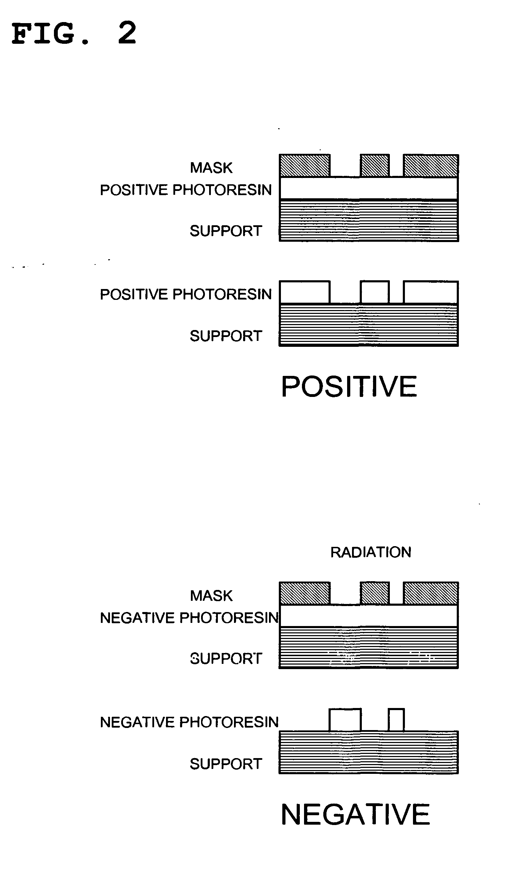

Fig. 2 shows schematically the behaviour of a positive photoresin and a negative photoresin when radiated with an electron/ light beam during a photolithographic process.
Fig. 3 is a block diagram of the operational units corresponding to a measuring device for organs transplant which uses a microelectrode according to the invention.

### Definitions

In the present invention, the term "transplant" means the moment of implanting, extraction and delivery.

In the present invention the term "negative photoresin" means any radiation-sensitive resin (to light or electron beam) where the radiated part polymerizes and the rest is ecthed away during development. The negative photoresin patterns the structure or boundary of the electrode.

In the present invention, the term "positive photoresin" means any radiation-sensitive resin (to light or electron beam) where the radiated areas soften or are damaged and are subsequently etched away during development, and where the non-radiated areas polymerizes by means of a heat treatment. Positive photoresin patterns the tracks and metal contacts over the substrate surface.

In the present invention, the term "bioimpedance" means the relation between the AC applied to a sample and the electric current obtained. When the sample belongs to an organism, the term "electric bioimpedance" term is often used, or simply "bio impedance".

The electric impedance values obtained depend on the sample geometry and its electrical properties. If the sample is composed of different elements, the values obtained depend strongly on the geometric relation between those elements.

Several physical and chemical properties of living tissue make them show a particular impedance that can be used to differentiate them and detect pathological states such as abnormal deviations.

Therefore, the microelectrode of the present invention can be applied to measure the bio impedance of any organ or tissue thus allowing to determine a change in its state.

### Detailed description of the invention

The present invention object is to provide a metallising preparation to obtain a microelectrode.

Therefore, according to the first element of the invention, the preparation can be used to obtain a microelectrode from a photopatterned polymer substrate with track and contacts over the surface, with a thickness greater than 20 µm.

The present invention overcomes the state of the art difficulties providing a process for obtaining a photopatterned polymeric substrate microelectrode, with the ductility required to prevent the microelectrode from breaking and the thickness required to avoid folding during the insertion, where said microelectrode is aimed to control organs or tissues during the transplant.

The metallization preparation, according to the first element of the invention, is characterized by that the microelectrode with metallic tracks and contacts on its surface obtained has a thickness in the 20 to 500 µm, generally from 150 to 400 and preferably from 250 to 350 µm.

The process comprises the following steps:
i) depositing over the support a layer of low adhesion material (low adhesion to the substrate) so it can be released in a later stage; the support can be any material, such as glass, and the low adhesion material can be, for instance, kapton;
ii) depositing, using spin coating, a negative photo resin A until the desired thickness is reached.
iii) patterning the design (boundary) of the negative photoresin substrate A using photolitography (light and heat treatment), without developing. Preferably, this substrate is shaped as a needle to facilitate its insertion in the organ to be transplanted, yet other shapes are possible.
iv) protecting the uncured areas of that substrate (negative photoresin A) using:
   - the deposition of a Cr/Au layer; followed by
   - the deposition, using photolitography, of a positive photoresin B, and
   - etch away the uncured positive photoresin B together with the Cr/Au layer from the cured surface of said substrate.
v) patterning the electrode design using the deposition of a second positive photoresin B' using photolitography.
vi) metallizing the resulting surface from the previous stage to create the sensing electrode area using, for instance, Ti/Pt.
vii) Etching away the positive photoresin polymers B and B' created in the previous steps, so a photopatterned polymeric substrate with electrode areas is obtained.
   Advantageously, with said step vii the metal layer area which is not part of the electrodes is etched away without any chemical etching of the metal, and subsequently,
viii) spin-coating a negative photoresin A patterned using ultraviolet light thus leaving those microelectrode areas designed to act as sensor uncovered; and finally
ix) performing the final development of the uncured polymeric areas of the negative photoresin A to obtain a microelectrode, and detach it from the support.

The negative photoresin A is preferably SU-8:

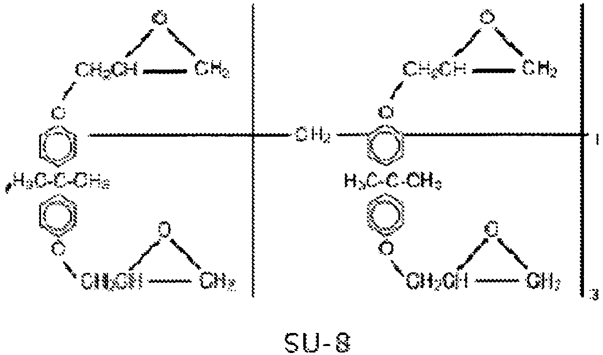

Positive photoresins B and B' mentioned in iv), v) and vii) steps can be the same or different. Said Positive photoresins B and B' are used to shape the electrodes. S1818 and SPR 220 are among the preferable, in which the resin used is Cresol Novolak, which is shown below. As any person skilled in the art knows, any other positive photoresin can be used in the process of the invention.

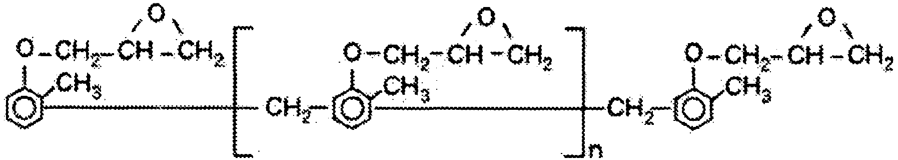

Advantageously, metals which etchers could be harmful for the structural material, i.e. for the substrate, can be used for the microelectrodes in said process, since it is not necessary to etch the metal, but only etch the positive B-B'. Thus, for example, etchers such as titanium and platinum, which are based on very harmful etchers like fluorhydric acid, can be avoided.

The utilization of the Cr/Au layer to avoid the cure of the polymer in the areas where it has to be removed by means of the development at the end of the process has to be done at a low temperature, always below 65 degrees centigrade, and with a minimum UV exposure, for example, by means of thermal evaporation or sputtering at 100 W with on/off cycles to prevent from excessive heating of the substrate. Chrome and gold are used as adhesion layer, because of their ductility, which prevents from "cracking" of the metallic layer in subsequent thermal processes.

The present invention also relates to the microelectrode obtained by means of the metallization process described above.

The microelectrode obtained can be used "in vivo", "in vitro" or "in situ" for a transplant organ, for impedance and or temperature measuring of said organ. Said microelectrode comprises a photopatternable polymeric substrate greater than 20 µm, preferably greater than 200 µm, and more preferably in the range from 150 to 400 µm, with tracks and contacts on its surface for the signal conditioning and treatment.

Advantageously, the microelectrode obtained overcomes the resistivity drawbacks of the Si substrates in certain frequencies.

In addition, the microelectrodes obtained have an specific application for measuring the impedance of an organ or tissue and, therefore, the device comprising said microelectrodes is specially suitable for the measurement of intratissular bioimpedance of organs and tissues.

A preferred application of microelectrodes is the monitoring of ischemia or ischemia-reperfusion of organs or tissue in animal experimentation. This is the situation that arises in an organ that is going to be transplanted. In these cases, the impedance is a marker of the tissular state. During ischemia or in a situation of lack of blood circulation, the cell runs out of oxygen and nutrients resulting in failures of the Na+/K+ membrane pump, thus causing the cell to absorb liquids (intracellular edema), and therefore the intercellular space to decrease. That is, there is an intracellular volume increase and an extracellular volume decrease. This is the relation monitorized by means of the bioimpedance. The bioimpedance increases with the intracelullar volume. When ischemia starts, the impedance increases due to intracellular edema. If this situation extends over time, the membrane finally breaks and the cell dies, thus causing the impedance to decrease.

In spite of the utilization of organs preservation techniques (preservative liquids, cold temperature, etc.) organs have a time limit for toleration of ischemia state: 4h for the heart, 8 for the liver, and 12 for the kidney. When this time limit is surpassed, the organ is no more considered viable. In spite of these protocols, the organ can resist more or less time depending on other factors such as age, related pathologies, genetic factors, etc. An easy and fast clinical method for predicting this does not exist yet. The surgeon decision regarding the implantation or not of the organ is based, at present, on the criterion of fulfilment of the protocols plus the microscopic visual aspect of the organ.

In these cases, bioimpedance can be an objective indicator of the organ evolution.

The invention is related as well to a device for analysing the temperature and/or impedance of at least one organ "in vivo", "in vitro" or "in situ" comprising:
- a microelectrode according to the invention;
- a signal conditioning module; and
- a signal treatment module.

Preferably, in this device the signal treatment module can be replaced by a telemetric module 8 to transmit the signal from outside the sterile container where the organ is placed to an external receiver. This external receiver could be, for example, a personal digital assistant.

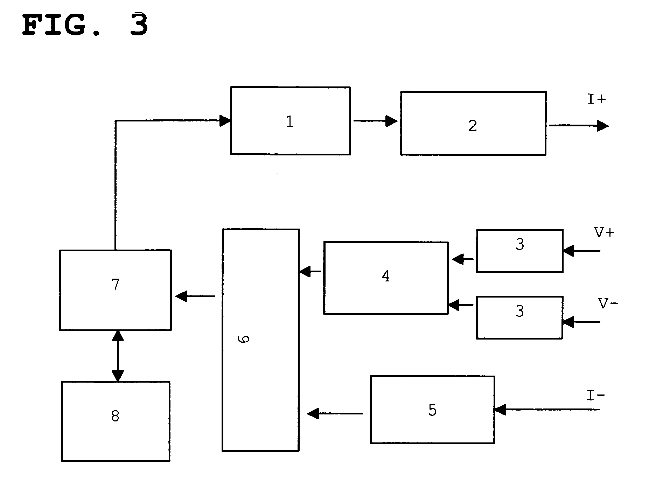

More specifically, according to a preferred embodiment, and referring to figure 3, said device comprises the following elements:
- A signal generator 1, consisting of a current or voltage source generating a multi or single-frequency signal.
- A signal conditioning that can be a low-pass filter conditioning the signal I+ to be applied to the load and which parameters depend on the frequency and impedance ranges to be measured and the specific application.
- A Buffer 3 for guaranteeing a minimum current leakage from the load to the circuitry, aimed to minimize the measurements errors.
- A differential amplifier 4 which amplifies and conditions the V+V- voltage signal of the load measured.
- A I to V converter 5 that measures the current I-flowing through the load.
- A demodulator 6, used to extract the real and imaginary part of the voltage V+V- and current I- signals, in order to be able to compute the impedance value. In this sense, two implementation options are considered:
   a. Digitizing (by means of a ADC converter) the voltage and current signals, and extract the real and imaginary part by multiplying the measured values by a sin and cos function in the CPU or
   b. Using an analogical demodulator and digitizing the gain and phase outputs (proportional to the module and phase of the load)
- A CPU 7 that controls the signal generator and calculates the impedance value starting from the voltage and current signals.

More preferably, the conditioning and telemetric module is a sterilizable, biocompatible and hermetic device (submerged in a preservative liquid) for medical use.

Next, a preferred embodiment for obtaining a microelectrode on a photopatternable polymeric substrate is disclosed.

### Preferred embodiment of the invention

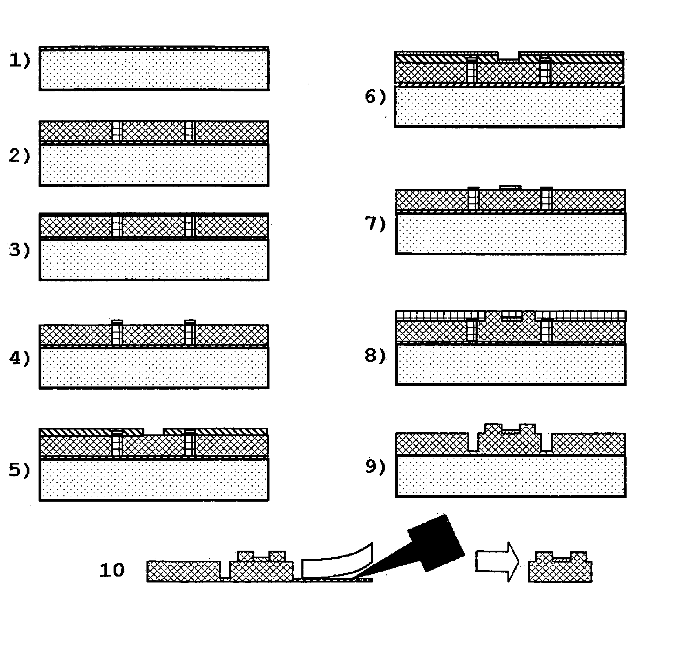

Obtaining of a microelectrode according to the metallization process defined in claim 1 and the following related to said metallization process (Figure 1)
1) A Kapton layer, for example 125 µm, is deposited over a glass support. In this way, the final removal of the electrode is easier. This step is only necessary in the case the substrate has to be detached from the support; Fig 1(1).
2) The necessary layers of SU-8 photopatternable polymer that are destined to be used as structural material for the device, for example up to 200 µm, are deposited by spin coating. Later, this material is illuminated and thermally treated to cure those parts destined to form the device; Fig. 1(2).
3) A Cr/Au layer, for example 50 µm of Cr and 150 µm of Au, is deposited to prevent from the curing of the polymer in the zones where said polymer has to be removed at the end of the process by development; Fig. 1(3).
4) Definition of the Cr/Au layer, for example 1.8 µm, by means of a photolitographic process through deposition of a positive photoresin B (for example: S1818), followed by an etching for protecting the non cured zone, but leaving the zone where the electrodes have to be deposited free; Fig. 1. (4).
5) Definition of a second positive photoresin B' (for example: SPR 220), i.e. 4 µm, that will be used to shape the electrodes; Fig. 1 (5).
6) Depositing the layer aimed to be used as electrodes, for example 50 µm of Ti and 150 µm of Pt; Fig .1 (6). This deposition must be done at a low temperature and with a minimum exposure to UV (for example, thermal evaporation or sputtering at 100 W by means of on/off cycles to prevent from excessive heating of the substrate).
7) Etch away the polymers with methanol; Fig. 1 (7). In this way, the metal layer which is not part of the electrodes is removed without need of etching (process known as lift-off).
8) A top layer of microelectrode structural material is deposited by spin coating, that is with negative photoresin SU-8, i.e. 20 µm, and is UV patterned leaving free only the sensing areas, which have a thickness of 10 µm, for example; Fig.1(8).
9) Final development of the structural material of the device; Fig. 1 (9).
10) Manual releasing of the device from the substrate, by means of a low adhesion material; Fig. 1 (10).

## Claims

1. Metallization process to obtain a microelectrode on a photopatternable polymeric substrate, **wherein** said substrate has a thickness greater than 20 µm with metallic contacts and tracks on its surface, said process comprising the following steps:
i) Depositing over the support a material with low adhesion with the substrate so it can be separated in a later stage;
ii) Depositing using spin coating a negative photoresin A until it reaches the desired thickness;
iii) Patterning the design (boundary) of the negative photoresin substrate A using photolitography (light and heat treatment), without developing;
iv) Protecting the uncured areas on that substrate by using the deposition of a Cr/Au layer, followed by the deposition of a positive photoresin B by means of a photolitographic process, and etch away the uncured positive photoresin B with the Cr/Au layer from the cured surface of the substrate;
v) Patterning the electrode design using the deposition of a second positive photoresin B' using photolitography;
vi) Metallizing the resulting surface from the previous stage to create the electrode areas using, for instance, Ti/Pt;
vii) Etching away the positive photoresin polymers B and B' created in the previous stages, so a photopatterned polymeric substrate with electrode areas is obtained;
viii) spin-coating a negative photoresin A patterned using ultraviolet light leaving uncovered those microelectrode sensing areas, and finally
ix) performing the final development of the uncured polymeric areas of the negative photoresin A to obtain a microelectrode and detach it from the support.

2. Metallization process according to claim 1, wherein said photopatternable polymer obtained from the negative photoresin is SU-8:

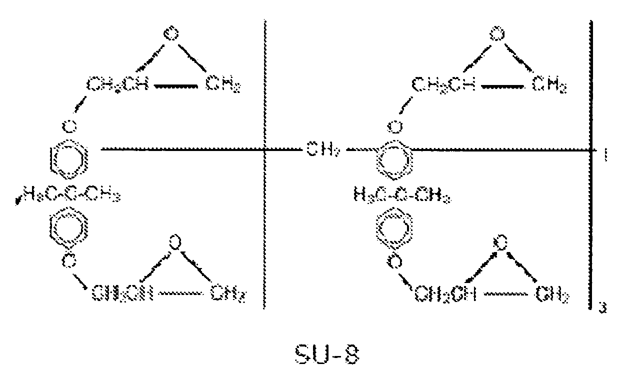

3. Metallization process according to claim 1, wherein said positive photoresins B y B' can be the same or different.

4. Metallization process according to claim 1, wherein in said step ii) a thickness greater than 200 µm is obtained.

5. Metallization process according to claim 1, wherein in said step iii) said substrate is patterned as a needle.

6. Microelectrode for insertion "in vivo", "in vitro" or "in situ" in an organ destined to transplant, for impedance and or temperature measuring of said organ, comprising a photopatternable polymeric substrate greater than 20 µm with tracks and contacts on its surface processed according to any of claims 1 to 5.

7. Microelectrode according to claim 6 wherein the photopatternable polymeric substrate is SU-8 with metallic tracks and contacts on its surface and a thickness from 150 to 400 µm.

8. Device for analysing the temperature and/or impedance of at least one organ "in vivo", "in vitro" or "in situ" comprising:
- a microelectrode according to claim 6 or 7;
- a signal conditioning module; and
- a signal treatment module.

9. Device for analysing the temperature and/or impedance of at least one organ during its transport comprising:
- a microelectrode according to claim 6 or 7;
- a signal conditioning module; and
- a telemetric module to transmit the signal from outside the sterile container where the organ is placed to an external receiver.
- Wherein the conditioning and telemetric module is a biocompatible hermetic sterilizable device for medical use.

10. Utilization of a device according to claim 8 or 9 for the monitorization of the intratissular temperature and bioimpedance of organs and tissues.

## Patentansprüche

1. Metallisierungsverfahren zur Herstellung einer Mikroelektrode auf einem fotogestaltbaren polymeren Substrat, wobei das Substrat eine Dicke von mehr als 20 µm mit metallischen Kontakten und Bahnen auf seiner Oberfläche aufweist, wobei das Verfahren die folgenden Schritte aufweist:
i) Ablagern über das Substrat eines Materials mit geringer Adhäsion zu dem Substrat, so dass es in einem späteren Stadium wieder getrennt werden kann;
ii) Aufbringen unter Verwendung einer Rotationsbeschichtung eines negativen Fotoharzes A bis es die gewünschte Dicke erreicht;
iii) Gestalten des Designs (Grenze) des negativen Fotoharzsubstrats A unter Verwendung von Fotolitografie (Licht- und Wärmebehandlung), ohne Entwicklung;
iv) Schützen der ungehärteten Bereiche auf dem Substrat unter Verwendung der Ablagerung einer Cr/Au-Schicht, gefolgt von der Ablagerung eines positiven Fotoharzes B mittels eines fotolitografischen Prozesses und Abätzen des ungehärteten positiven Fotoharzes mit der Cr/Au-Schicht von der gehärteten Oberfläche des Substrats;
v) Gestalten des Elektrodendesigns unter Verwendung der Ablagerung eines zweiten positiven Fotoharzes B' unter Verwendung von Fotolitografie;
vi) Metallisieren der sich ergebenden Oberfläche aus dem vorhergehenden Stadium, um die Elektrodenbereiche zu erzeugen, unter Verwendung beispielsweise von Ti/Pt;
vii) Abätzen der positiven Fotoharzpolymere B und B', die in den vorhergehenden Stadien erzielt wurden, so dass ein fotogestaltetes Polymersubstrat mit Elektrodenbereichen erzielt wird;
viii) Rotationsbeschichten eines negativen Fotoharzes A, das unter Verwendung von ultraviolettem Licht gestaltet ist, unter Unbedecktlassen jener Mikroelektrodenerfassungsbereiche, und schließlich
ix) Durchführen der finalen Entwicklung der ungehärteten polymerischen Bereiche des negativen Fotoharzes A, um eine Mikroelektrode zu erhalten und Lösen von dieser von der Unterlage.

2. Metallisierungsverfahren nach Anspruch 1, wobei das fotogestaltbare Polymer, erhalten von dem negativen Fotoharz gleich SU-8 ist:

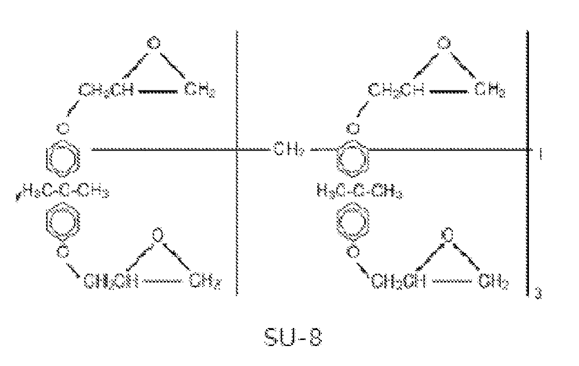

3. Metallisierungsverfahren nach Anspruch 1, wobei die positiven Fotoharze B y B' dieselben oder unterschiedlich sein können.

4. Metallisierungsverfahren nach Anspruch 1, wobei in dem Schritt ii) eine Dicke größer als 200 µm erhalten wird.

5. Metallisierungsverfahren nach Anspruch 1, wobei in dem Schritt iii) das Substrat als eine Nadel ausgebildet ist.

6. Mikroelektrode für die Einführung "in vivo", "in vitro" oder "in situ" in ein Organ, das zum Transplantieren bestimmt ist, zum Messen der Impedanz oder der Temperatur des Organs, mit einem fotogestaltbaren Polymersubstrat größer als 20 µm mit Bahnen und Kontakten auf seiner Oberfläche, hergestellt nach einem der Ansprüche 1 bis 5.

7. Mikroelektrode nach Anspruch 6, wobei das fotogestaltbare polymerische Substrat SU-8 ist, mit metallischen Bahnen und Kontakten auf seiner Oberfläche und einer Dicke von 150 bis 400 µm.

8. Vorrichtung zum Analysieren der Temperatur und/oder Impedanz wenigstens eines Organs "in vivo", "in vitro" oder "in situ", die aufweist:
- eine Mikroelektrode nach Anspruch 6 oder 7;
- einem Signalkonditioniermodul; und
- einem Signalverarbeitungsmodul.

9. Vorrichtung zum Analysieren der Temperatur und/oder Impedanz wenigstens eines Organs, während seines Transports, die aufweist:
- eine Mikroelektrode nach Anspruch 6 oder 7;
- ein Signalkonditioniermodul; und
- ein telemetrisches Modul, um das Signal von außerhalb des sterilen Containers, in dem sich das Organ befindet, zu einem äußeren Empfänger zu übertragen,
- wobei das Konditionier- und telemetrische Modul eine biokompatible hermetische sterilisierbare Vorrichtung für die medizinische Verwendung ist.

10. Verwendung einer Vorrichtung nach Anspruch 8 oder 9 für die Überwachung der Intragewebs-Temperatur und -Bioimpedanz von Organen und Geweben.

## Revendications

1. Procédé de métallisation pour obtenir une microélectrode sur un substrat polymère photomodelable, dans lequel ledit substrat a une épaisseur supérieure à 20 µm avec des contacts métalliques et des pistes sur sa surface, ledit procédé comprenant les étapes suivantes :
i) déposer sur le support un matériau à faible adhérence avec le substrat de manière à pouvoir être séparé à un stade ultérieur ;
ii) déposer par revêtement par centrifugation une photorésine négative A jusqu'à ce qu'elle atteigne l'épaisseur voulue ;
iii) former le dessin (contour) du substrat de photorésine négative A par photolithographie (traitement à la lumière et à la chaleur), sans développement ;
iv) protéger les zones non durcies sur ce substrat en utilisant le dépôt d'une couche de Cr/Au, suivi par le dépôt d'une photorésine positive B, au moyen d'un procédé photolithographie, et éliminer par attaque chimique la photorésine positive B non durcie avec la couche de Cr/Au de la surface durcie du substrat ;
v) former le dessin d'électrode en utilisant le dépôt d'une seconde photorésine positive B' par photolithographie ;
vi) métalliser la surface obtenue à l'étape précédente pour créer les zones d'électrode en utilisant, par exemple, du Ti/Pt ;
vii) éliminer par attaque chimique les polymères de photorésine positive B et B' créés dans les étapes précédentes, de manière à obtenir un substrat polymère photomodelé avec des zones d'électrode ;
viii) déposer par revêtement par centrifugation une photorésine négative A modelée en utilisant de la lumière ultraviolette laissant à découvert ces zones de détection de microélectrode, et enfin
ix) réaliser le développement final des zones polymères non durcies de la photorésine négative A pour obtenir une microélectrode et la détacher du support.

2. Procédé de métallisation selon la revendication 1, dans lequel ledit polymère photomodelable obtenu à partir de la photorésine négative est du SU-8 :

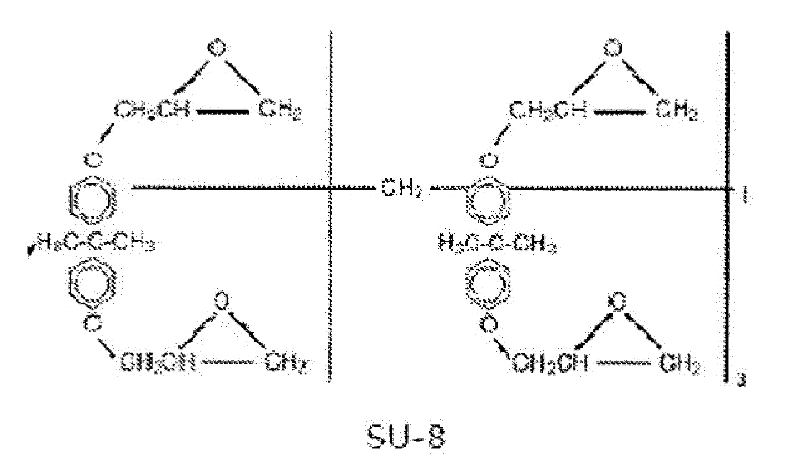

3. Procédé de métallisation selon la revendication 1, dans lequel lesdites photorésines positives B et B' peuvent être identiques ou différentes.

4. Procédé de métallisation selon la revendication 1, dans lequel dans ladite étape ii) une épaisseur supérieure à 200 µm est obtenue.

5. Procédé de métallisation selon la revendication 1, dans lequel dans ladite étape iii) ledit substrat est modelé sous la forme d'une aiguille.

6. Microélectrode pour insertion « in vivo », « in vitro » ou « in situ » dans un organe destiné à une transplantation, pour une mesure de l'impédance et/ou de la température dudit organe, comprenant un substrat polymère photomodelable supérieur à 20 µm avec des pistes et des contacts sur sa surface traitée conformément à l'une des revendications 1 à 5.

7. Microélectrode selon la revendication 6, dans laquelle le substrat polymère photomodelable est du SU-8 avec des pistes métalliques et des contacts sur sa surface et une épaisseur allant de 150 à 400 µm.

8. Dispositif pour analyser la température et/ou l'impédance d'au moins un organe « in vivo », « in vitro » ou « in situ » comprenant :
- une microélectrode selon la revendication 6 ou 7 ;
- un module de conditionnement du signal ; et
- un module de traitement du signal.

9. Dispositif pour analyser la température et/ou l'impédance d'au moins un organe pendant son transport, comprenant :
- une microélectrode selon la revendication 6 ou 7 ;
- un module de conditionnement du signal ; et
- un module télémétrique pour transmettre le signal de l'extérieur du récipient stérile où l'organe est placé vers un récepteur externe.
- Dans lequel le module de conditionnement et télémétrique est un dispositif stérilisable hermétique biocompatible à usage médical.

10. Utilisation d'un dispositif selon la revendication 8 ou 9 pour la surveillance de la température intratissulaire et de la bio-impédance d'organes et de tissus.
